(19) Europäisches Patentamt
European Patent Office
Office européen des brevets

(11) Veröffentlichungsnummer: **0 487 980 A1**

(12) # EUROPÄISCHE PATENTANMELDUNG

(21) Anmeldenummer: **91119397.7**

(51) Int. Cl.5: **C07D 263/06**, C07D 413/06

(22) Anmeldetag: **14.11.91**

(30) Priorität: **24.11.90 DE 4037437**

(43) Veröffentlichungstag der Anmeldung: **03.06.92 Patentblatt 92/23**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Wagner, Adalbert, Dr.**
**Kleiststrasse 9**
**W-6238 Hofheim am Taunus(DE)**
Erfinder: **Henning, Rainer, Dr.**
**Im Höhlchen 16**
**W-6234 Hattersheim am Main(DE)**
Erfinder: **Nickel, Wolf-Ulrich, Dr.**
**Sportplatzstrasse 2a**
**W-6238 Hofheim am Taunus(DE)**

(54) **Aminodiol-Derivate.**

(57) Die Erfindung betrifft Verbindungen der Formel

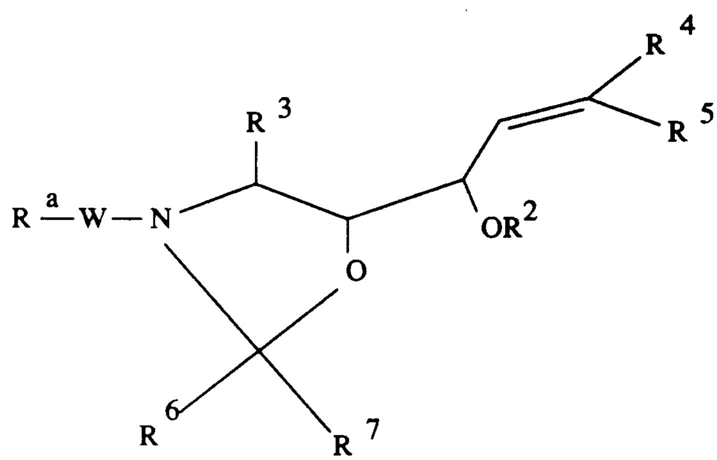

worin W für -CO-, -O-CO-, $-SO_2-$ oder -NH-CO- steht, $R^a$, $R^2$, $R^4$, $R^5$, $R^6$ und $R^7$ für Wasserstoff oder einen organischen Rest stehen und $R^3$ Alkyl, Cycloalkyl oder Cycloalkylalkyl bedeutet, Verfahren zu deren Herstellung, Zwischenprodukte sowie deren Verwendung bei der Herstellung von Inhibitoren des Renins und der HIV-Protease.

EP 0 487 980 A1

Die Erfindung betrifft Verbindungen der Formel Ia bzw. Ib

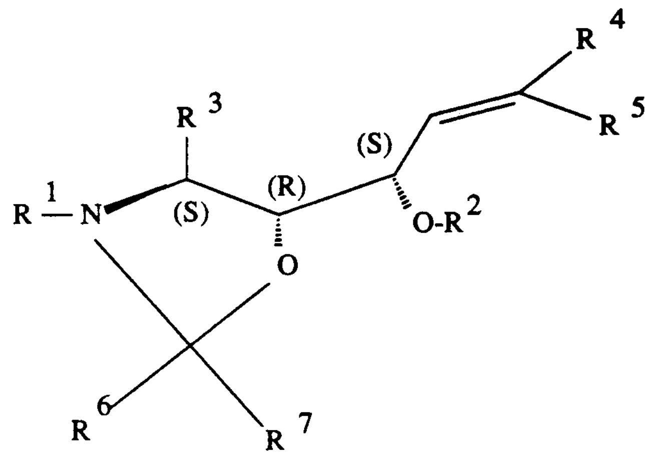

(Ia)

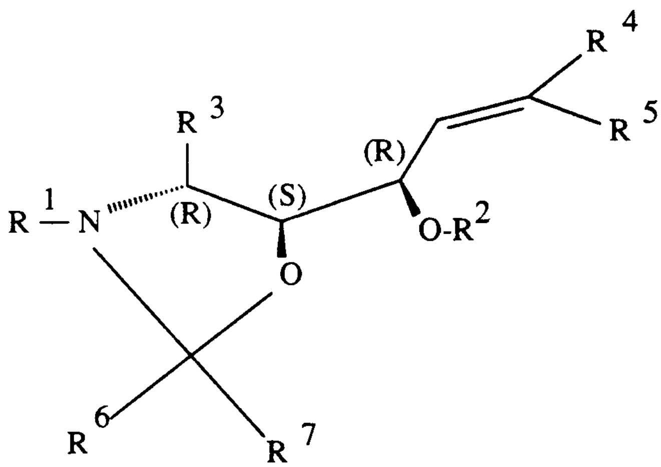

(Ib)

sowie deren Salze, in welcher $R^1$ $R^a$-W- bedeutet;
W für -CO-, -O-CO-, $SO_2$- oder -NH-CO- steht;
$R^a$ für Wasserstoff, $(C_1$-$C_{10})$-Alkyl, das gegebenenfalls ein- oder zweifach ungesättigt ist und das gegebenenfalls mit bis zu 3 gleichen oder verschiedenen Resten aus der Reihe Hydroxy, $(C_1$-$C_7)$-Alkoxy, $(C_1$-$C_7)$-Alkanoyloxy, Carboxy, $(C_1$-$C_7)$-Alkoxycarbonyl, Halogen, Amino, $(C_1$-$C_7)$-Alkylamino, Di-$(C_1$-$C_7)$-alkylamino, $(C_1$-$C_5)$-Alkoxycarbonylamino, $(C_7$-$C_{15})$-Aralkoxycarbonylamino substituiert ist, $(C_3$-$C_8)$-Cycloalkyl, $(C_3$-$C_8)$-Cycloalkyl-$(C_1$-$C_6)$-alkyl, $(C_6$-$C_{14})$-Aryl, das gegebenenfalls durch einen oder zwei gleiche oder verschiedene Reste aus der Reihe F, Cl, Br, J, Hydroxy, $(C_1$-$C_7)$-Alkoxy, $(C_1$-$C_7)$-Alkyl, $(C_1$-$C_7)$-Alkoxycarbonyl, Amino, gegebenenfalls mit bis zu 2 Halogenen substituiertes Anilino und Trifluormethyl substituiert ist, $(C_6$-$C_{14})$-Aryl-$(C_1$-$C_6)$-alkyl, worin der Arylteil gegebenenfalls durch einen oder zwei gleiche oder verschiedene Reste aus der Reihe F, Cl, Br, J, Hydroxy, $(C_1$-$C_7)$-Alkoxy, $(C_1$-$C_7)$-Alkyl, $(C_1$-$C_7)$-Alkoxycarbonyl, Amino, $(C_1$-$C_7)$-Alkylamino, Di-$(C_1$-$C_7)$-alkylamino, Carboxy, Carboxymethoxy, Amino-$(C_1$-$C_7)$-alkyl, $(C_1$-$C_7)$-Alkylamino-$(C_1$-$C_7)$-alkyl, Di-$(C_1$-$C_7)$-alkylamino-$(C_1$-$C_7)$-alkyl, $(C_1$-$C_7)$-Alkoxycarbonylmethoxy, Carbamoyl, Sulfamoyl, $(C_1$-$C_7)$-Alkoxysulfonyl und Sulfo substituiert ist, oder den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom als Ringglieder, der gegebenenfalls durch einen oder mehrere Reste aus der Reihe F, Cl, Br, Hydroxy, $(C_1$-$C_7)$-Alkoxy, $(C_1$-$C_7)$-Alkyl, $(C_1$-$C_8)$-Alkoxycarbonyl, Amino und Trifluormethyl mono-, di- oder trisubstituiert ist, steht.

$R^2$ ist H oder eine Schutzgruppe der Hydroxylgruppe, wie sie z. B. in T.W. Greene, "Protective Groups in Organic Synthesis"; A. Wiley-Interscience Publications; New York-Chichester-Brisbane-Toronto-Singapore, 1981 beschrieben sind. Insbesondere kommen davon Benzyl, p-Methoxybenzyl, Methoxymethyl, Methoxyethyl, Benzyloxymethyl, Acetyl, Trimethylsilyl, tert.-Butyldimethylsilyl und tert.-Butyldiphenylsilyl in Frage.

$R^3$ bedeutet ($C_1$-$C_{12}$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_6$)-alkyl, wobei der Cycloalkylteil jeweils gegebenenfalls mit ($C_1$-$C_6$)-Alkyl substituiert ist.

$R^4$ und $R^5$ sind gleich oder verschieden und bedeuten Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl-($C_1$-$C_6$)-alkyl, wobei Aryl jeweils mit ein, zwei oder drei gleichen oder verschiedenen Resten aus der Gruppe ($C_1$-$C_6$)-Alkyl und Halogen substituiert sein kann, Het oder Het-($C_1$-$6_6$)-alkyl, wobei Het für einen 5-, 6- oder 7-gliedrigen heterocyclischen Ring steht, der gegebenenfalls benzanelliert ist und aromatisch, teilhydriert oder vollständig hydriert sein kann und der als Heteroelement ein oder zwei gleiche oder verschiedene Reste aus der Gruppe N, O, S, NO, SO und $SO_2$ enthält und der mit ein oder zwei gleichen oder verschiedenen Resten aus der Gruppe ($C_1$-$C_6$)-Alkyl, ($C_1$-$C_4$)-Alkoxy und Halogen substituiert sein kann.

$R^6$ und $R^7$ sind gleich oder verschieden und bedeuten Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl-($C_1$-$C_6$)-alkyl, wobei Aryl jeweils mit ein, zwei oder drei gleichen oder verschiedenen Resten aus der Gruppe ($C_1$-$C_6$)-Alkyl, ($C_1$-$C_4$)-Alkoxy und Halogen substituiert sein kann; oder $R^6$ und $R^7$ bedeuten mit dem sie tragenden Kohlenstoffatom ($C_3$-$C_{12}$)-Cycloalkyl. Die Verbindungen der Formeln Ia und Ib sind Enantiomere. Die Erfindung betrifft sowohl die optisch reinen Verbindungen als auch deren Stereoisomerengemische, wie Racemate.

Alkyl kann geradkettig oder verzweigt sein. Das gleiche gilt für davon abgeleitete Reste wie Alkoxy.

Unter Cycloalkyl versteht man z. B. Cyclopropyl, Cyclobutyl, Cyclopentyl, Cyclohexyl oder Cycloheptyl.

Ein als Het bezeichneter Rest hat vorzugsweise eine der folgenden Bedeutungen: Pyridyl, Thiazolyl, Thienyl, Pyranyl, Benzofuryl, Isobenzofuryl, Furyl, Pyrrolyl, Imidazolyl, Pyrazinyl, Indazolyl, Chinolyl, Isochinolyl, Phthalazinyl, Pyrimidinyl, Pyrazinyl, Indolizinyl, Isoindolyl, Indolyl, Chinoxalinyl, Chinazolinyl, Cinnolinyl, Oxazolyl, Isoxazolyl, Isothiazolyl. Die Reste können aromatisch, teilhydriert oder vollständig hydriert sein. Sie können mit einem oder zwei gleichen oder verschiedenen Resten aus der Gruppe ($C_1$-$C_6$)-Alkyl, ($C_1$-$C_4$)-Alkoxy und Halogen substituiert sein.

Unter ($C_6$-$C_{14}$)-Aryl versteht man beispielsweise Phenyl, Naphthyl oder Biphenylyl; bevorzugt ist Phenyl.

Halogen ist Fluor, Chlor, Brom oder Jod.

Als Salze kommen insbesondere Salze mit Mineralsäuren, wie HCl, $H_2SO_4$, $H_3PO_4$ in Frage, in die die Verbindungen der Formel Ia bzw. Ib z. B. zum Zwecke der Lagerung überführt werden.

Bevorzugt sind Verbindungen der Formel Ia bzw. Ib, in welcher

W wie oben definiert ist,

$R^a$ für Wasserstoff, ($C_1$-$C_{10}$)-Alkyl, das gegebenenfalls ein- oder zweifach ungesättigt ist, ($C_3$-$C_8$)-Cycloalkyl, ($C_3$-$C_8$)-Cycloalkyl-($C_1$-$C_6$)-alkyl, ($C_6$-$C_{14}$)-Aryl, ($C_6$-$C_{14}$)-Aryl-($C_1$-$C_6$)-alkyl, oder für den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom auch als Ringglieder steht,

$R^2$ wie oben definiert ist.

$R^3$ ($C_1$-$C_6$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_3$)-alkyl bedeutet;

$R^4$ und $R^5$ wie oben definiert sind; und

$R^6$ und $R^7$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl bedeuten; oder diese zusammen mit dem sie tragenden Kohlenstoffatom für ($C_3$-$C_{12}$)-Cycloalkyl stehen;

sowie deren Salze.

Besonders bevorzugt sind Verbindungen der Formel Ia bzw. Ib, in welcher W für -CO-, -O-CO- steht und $R^a$ Wasserstoff, ($C_1$-$C_{10}$)-Alkyl, das gegebenenfalls ein- oder zweifach ungesättigt ist, ($C_3$-$C_8$)-Cycloalkyl, ($C_3$-$C_8$)-Cycloalkyl-($C_1$-$C_6$)-alkyl, ($C_6$-$C_{14}$)-Aryl, ($C_6$-$C_{14}$)-Aryl-($C_1$-$C_6$)-alkyl, oder den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom als Ringglieder bedeutet;

$R^2$ wie oben definiert ist; und

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;

$R^4$ und $R^5$ wie oben definiert sind; und

$R^6$ und $R^7$ gleich sind und Wasserstoff oder ($C_1$-$C_4$)-Alkyl bedeuten, oder diese zusammen mit dem sie tragenden Kohlenstoffatom für ($C_5$-$C_7$)-Cycloalkyl stehen;

sowie deren Salze.

Ganz besonders bevorzugt sind Verbindungen der Formel Ia bzw. Ib, in welcher
$R^1$, $R^2$ wie oben definiert sind,
$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;
$R^4$ und $R^5$ gleich oder verschieden sind und Wasserstoff, $(C_1$-$C_4)$-Alkyl, $(C_5$-$C_7)$-Cycloalkyl, $(C_5$-$C_7)$-Cycloalkyl-$(C_1$-$C_3)$-alkyl, Phenyl, Benzyl, 2-Pyridyl, 3-Pyridyl, 4-Pyridyl, 2-Thiazolyl, 4-Thiazolyl, 5-Thiazolyl, 2-Thienyl, 3-Thienyl, 1-Methylimidazol-2-yl, 1-Methylimidazol-4-yl oder 1-Methylimidazol-5-yl bedeuten; und
$R^6$ und $R^7$ gleich sind und Wasserstoff, $(C_1$-$C_4)$-Alkyl oder zusammen mit dem sie tragenden Kohlenstoffatom ein $(C_5$-$C_7)$-Cycloalkyl bedeuten,
sowie deren Salze.

Die Erfindung betrifft weiterhin ein Verfahren zur Herstellung von Verbindungen der Formel Ia bzw. Ib, das dadurch gekennzeichnet ist, daß man eine Verbindung der Formel IIa bzw. IIb

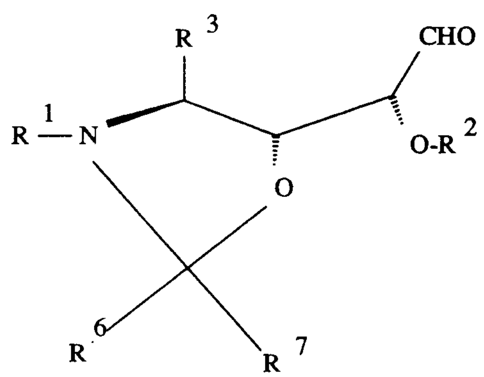

(IIa)

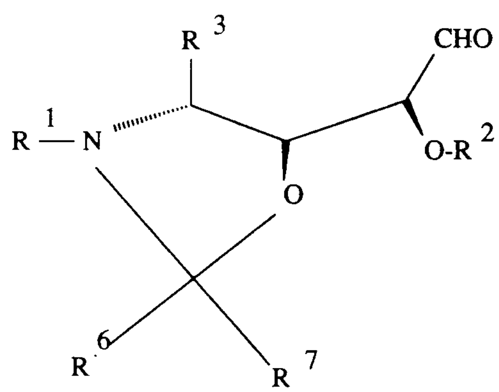

(IIb)

in welcher die Reste $R^1$, $R^2$, $R^3$, $R^6$ und $R^7$ wie oben definiert sind, umsetzt mit einem Phosphoran der Formel III,

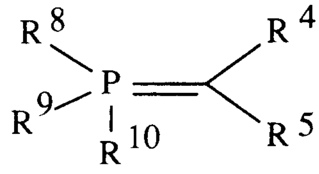

(III)

in welcher $R^4$ und $R^5$ wie oben definiert sind und $R^8$, $R^9$ und $R^{10}$ für gleiche oder verschiedene Arylreste, vorzugsweise Phenyl steht.

Zur Herstellung einer Verbindung der Formel IIa bzw. IIb kann man von geeignet geschützten Aminosäurederivaten ausgehen. Diese liefern IIa bzw. IIb durch zweimalige Umsetzung mit dem nucleophilen Formyläquivalent 2-Trimethylsilyl-thiazol TST (Schema 1) (A. Dondoni et al., J. Org. Chem. 1990, 55,

1439) in einem gegen dieses Nucleophil inerten Lösungsmittel wie Diethylether, Di-n-butylether, MTB, Dichlormethan, DIP, THF, Tetrahydropyran oder DME bei einer Temperatur zwischen -40 ° C und dem Siedepunkt des Lösungsmittels, bevorzugt zwischen -40 ° C und +25 ° C. Die erste Addition von TST erfolgt mit hoher syn-Selektion, die zweite mit hoher anti-Selektion. Die entstehende Hydroxyfunktion wird im nächsten Reaktionsschritt unter Verwendung literaturbekannter Methoden geschützt. Die Freisetzung der Formylgruppe kann durch folgende Reaktionssequenz durchgeführt werden:

1) N-Alkylierung mit einem geeigneten Alkylierungsreagenz wie z. B. Dimethylsulfat oder Methyljodid ohne Lösungsmittel oder in einem geeigneten inerten Lösungsmittel wie z. B. Acetonitril, THF, MTB, DIP, EE bei Temperaturen zwischen 25 ° C und dem Siedepunkt des entsprechenden Lösungsmittels.

2) Reduktion mit einem hydridübertragenden Reagenz wie z. B. $NaBH_4$ in einem für diesen Reaktionstypen geeigneten Lösungsmittel wie z. B. Methanol, Ethanol bei Temperaturen zwischen -30 ° C und 25 ° C, bevorzugt zwischen -20 ° C und 0 ° C.

3) Freisetzung der Aldehydgruppe durch Einwirkung von Reagenzien zur Spaltung von Thioacetalen (siehe Tamura et al., Synthesis 312 (1973), Seebach et al., ibid. 357 (1977) und dort zitierte Literatur), bevorzugt durch N-Bromsuccinimid in einem Gemisch aus Aceton und Wasser bevorzugt bei 0 ° C bis 30 ° C.

Schema 1

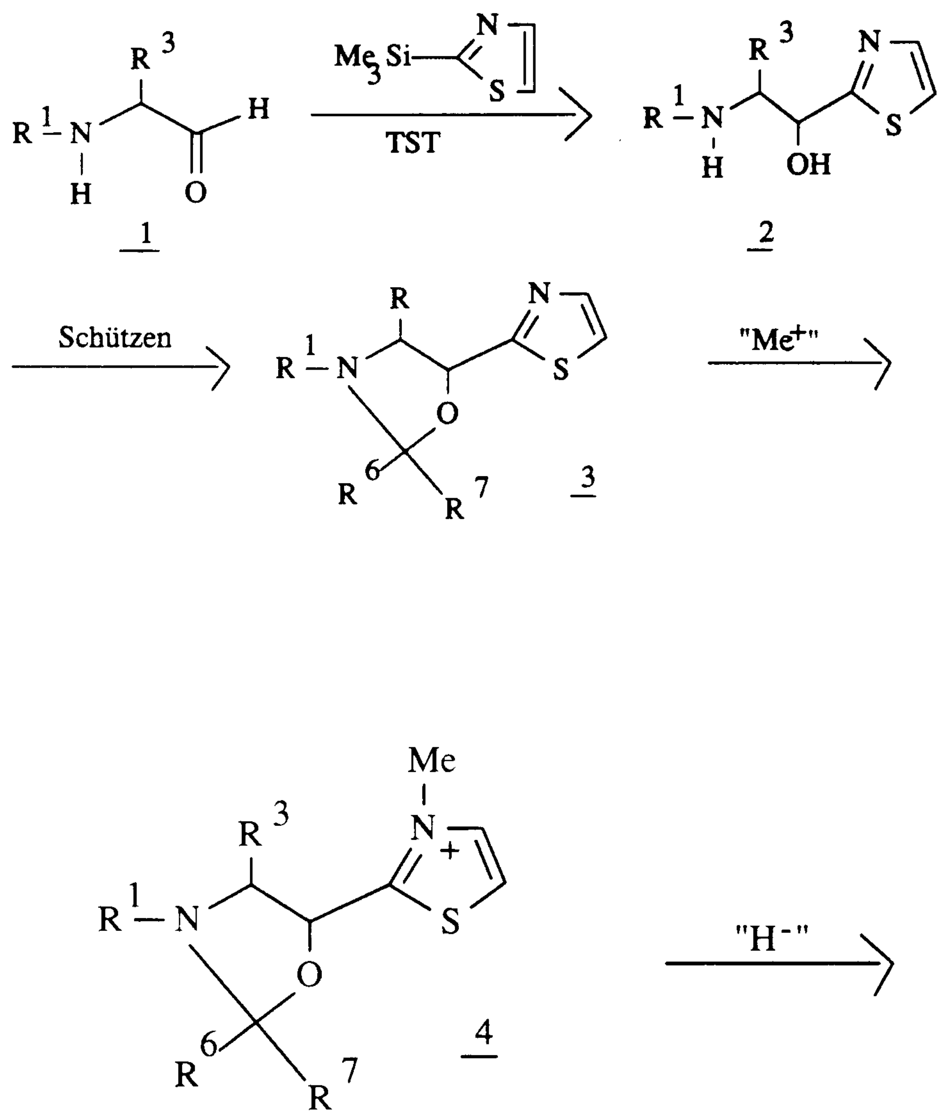

1 2 3 4

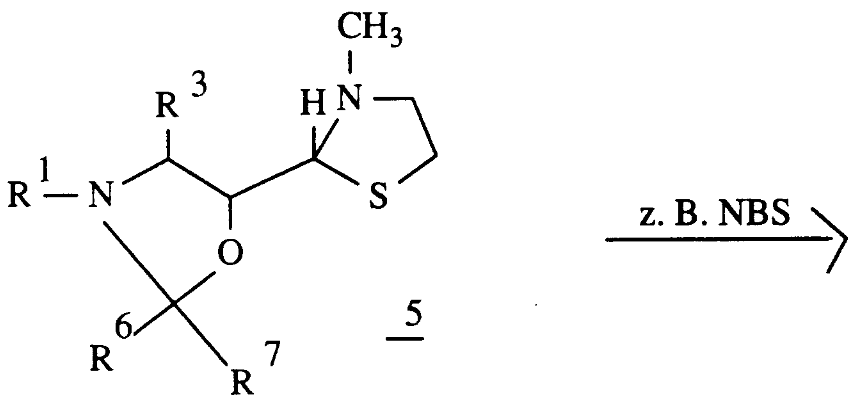


5

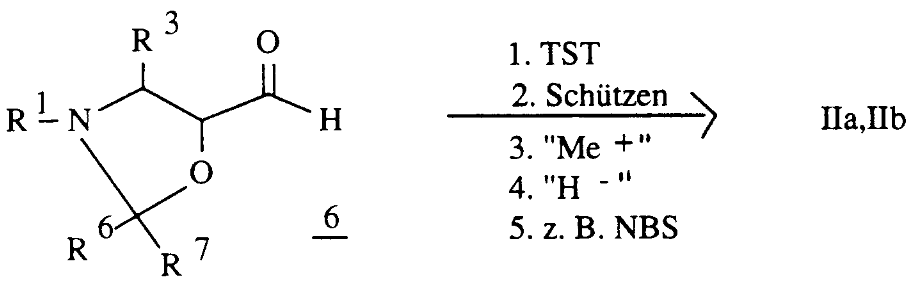


6

Die Wittig-Reaktion mit einem geeigneten Phosphoran in einem inerten Lösungsmittel wie Diethylether, Di-n-butylether, MTB, DIP, THF, DME, Dioxan bei -30 °C bis zum Siedepunkt des Lösungsmittels, bevorzugt zwischen 0 °C und 30 °C liefert die Titelverbindungen der Formel Ia bzw. Ib. Ebenso kann man den Aldehyd der Formel IIa bzw. IIb mit dem Anion eines geeigneten Phosphinoxids unter Bildung der Titelverbindung umsetzen (Horner; siehe Krauch, Kunz, Reaktionen der Organischen Chemie, Hüthig Verlag Heidelberg 1976, Seite 241).

Die erfindungsgemäßen Verbindungen der Formel Ia und Ib sind wertvolle Zwischenprodukte bei der Herstellung von Pharmazeutika, insbesondere von Inhibitoren des Renins und der HIV-Protease. Inhibitoren, bei deren Synthese diese Verbindungen vorteilhaft verwendet werden können, sind z. B. in FEBS Lett. Bd. 230, 38 (1988), J. Med. Chem. Bd. 31, 2264 (1988), ibid. 2277, Biochem. Biophys. Res. Commun. Bd. 146, 959 (1987) und EP-A-370 454 beschrieben.

Verzeichnis der verwendeten Abkürzungen:

| | |
|---|---|
| DC | Dünnschichtchromatographie |
| DCI | Desorption Chemical Ionisation |
| DIP | Diisopropylether |
| DME | 1,2-Dimethoxyethan |
| EE | Essigsäureethylester |
| FAB | Fast atom bombardment |
| Hep | n-Heptan |
| M | Molekularpeak |
| MeOH | Methanol |
| MS | Massenspektrum |
| MTB | Methyl-tert.-butylether |
| $Et_2O$ | Diethylether |
| R.T. | Raumtemperatur |
| Schmp. | Schmelzpunkt |
| THF | Tetrahydrofuran |
| NBS | N-Bromsuccinimid |
| TST | Trimethylsilylthiazol |

Red-Al Natrium-bis-(2-methoxyethoxy)-dihydroaluminat
$CH_2Cl_2$ Dichlormethan

Die nachstehenden Beispiele dienen zur Erläuterung der vorliegenden Erfindung, ohne daß diese darauf beschränkt wäre:

Beispiel 1

(2S,1RS)-2-t-Butyloxycarbonylamino-3-cyclohexyl-1-(2-thiazolyl)-propanol

3,14 g (10 mmol) N-Boc-L-Cyclohexylalanin-N,O-dimethylamid werden in 30 ml trockenem THF gelöst und auf 0°C gekühlt. 5,7 ml (20 mmol) Red-Al (70 % in Toluol) werden zugetropft. Nach 2 Std. Rühren bei 0°C wird auf 200 ml Eiswasser gegossen und 3 x mit Essigester extrahiert. Die vereinigten organischen Phasen werden mit gesättigter NaCl-Lösung gewaschen, mit $Na_2SO_4$ getrocknet und eingeengt. Man erhält 2,6 g N-Boc-L-Cyclohexylalaninal, das als Rohprodukt in 30 ml trockenem $CH_2Cl_2$ gelöst wird und bei -30°C tropfenweise mit 3,13 g (20 mmol) 2-Trimethylsilylthiazol versetzt wird. Der Ansatz wird im Tiefkühlschrank bei -25°C 14 Stunden aufbewahrt. Nach Einengen wird in 30 ml THF/5 ml $H_2O$ gelöst und mit 3 g (20 mmol) Cäsiumfluorid versetzt. Nach 7 Stunden Rückfluß wird mit Essigester verdünnt und 2 x mit ges. NaCl-Lösung gewaschen. Nach Trocknen über $Na_2SO_4$ wird eingeengt.

Das Produkt wird über Kieselgel (Laufmittel EE/Hep 1 : 2) von geringen Mengen nicht umgesetztem Aldehyd abgetrennt. Man erhält 1,83 g (54 %) der Titelverbindung. Sie ist laut NMR ein 5 : 1-Gemisch (R- bzw. S-Konfiguration am Kohlenstoff C-1).
$R_f$ (EE/Hep 1/1) = 0,21; MS (DCl) = 341 (M + 1).

Beispiel 1a

(4S,5R)-3-t-Butyloxycarbonyl-4-cyclohexylmethyl-2,2-dimethyl-5-(2-thiazolyl)-oxazolidin

23 g der Titelverbindung 1 (67,6 mmol) werden in 250 ml Toluol mit 40 ml 2,2-Dimethoxypropan und 2,5 g p-Toluolsulfonsäure 48 Stunden am Rückfluß gekocht. Nach Abkühlen wird mit 1 N $NaHCO_3$-Lösung gewaschen, mit $MgSO_4$ getrocknet und eingeengt. Das Rohprodukt wird an $SiO_2$ mit EE/Cyclohexan (1 : 4) gereinigt. Man erhält 12,5 g der Titelverbindung.
$R_f$ (EE/Hep 1/1) = 0,55; MS (DCl) = 381 (M + 1); Schmp. = 89 - 91°C,
$[\alpha]^{22}_D$ = -67,9° (c = 1, $CH_3OH$).

Beispiel 1b

(4S,5R,1R)-3-t.-Butyloxycarbonyl-4-cyclohexylmethyl-2,2-dimethyl-5-[1'-hydroxy-1'-(2-thiazolyl)-methyl]-oxazolidin

12,5 g (32,8 mmol) der Titelverbindung 1a werden in 70 ml Acetonitril gelöst. 3,9 ml (1,25 Äquivalente) Dimethylsulfat werden zugegeben und die Mischung 7 7 Stunden zum Sieden erhitzt. Nach Stehen über Nacht bei RT wird zur Trockne eingeengt. Der feste Rückstand wird in 150 ml Methanol gelöst. 4,3 g (0,013 Mol) Natriumborhydrid werden unter guter Kühlung portionsweise zugegeben, wobei die Temperatur nicht über -10°C steigen soll. Nach 30 Minuten Nachrühren bei 0°C werden 40 ml Aceton zugegeben. Nach Einengen wird in 500 ml EE aufgenommen und mit 200 ml ges. NaCl-Lösung gewaschen. Nach Trocknen mit $Na_2SO_4$ wird eingeengt und das Rohprodukt durch Filtration über wenig Kieselgel grob gereinigt, wobei 14 g anfallen (Verbindung 5, Schema 1). Diese werden in 250 ml Aceton/$H_2O$ (95 : 5) gelöst, 16,5 g NBS werden unter Kühlung (≦ 25°C) in 10 min zugegeben. Nach Ende der Zugabe wird stark gekühlt und in kleinen Portionen mit 500 ml ges. $Na_2SO_3$-Lösung versetzt (≦ 25°C, stark exotherm!). Nach Verdünnen mit 500 ml $H_2O$ wird 3 x mit $Et_2O$ extrahiert. Die vereinigten Extrakte werden 2 x mit $H_2O$ und 1 x mit gesättigter NaCl-Lösung gewaschen, mit $Na_2SO_4$ getrocknet und eingeengt. Durch azeotrope Destillation am ®Rotavapor mit Toluol werden Wasserreste entfernt. Man erhält 5,9 g Aldehyd (Verbindung 6, Schema 1), der unmittelbar in 80 ml $CH_2Cl_2$ gelöst und auf -40°C gekühlt wird. 5,88 g (37,4 mmol) Trimethylsilylthiazol werden zugespritzt. Die Mischung wird über Nacht langsam auf RT aufgewärmt. Nach Einengen wird in 80 ml THF aufgenommen und mit 11,8 g (37,4 mmol) Tetrabutylammoniumfluorid-Trihydrat versetzt. Nach 2 Stunden wird mit EE verdünnt, 2 x mit $H_2O$ und 1 x mit ges. NaCl-Lösung gewaschen, mit $Na_2SO_4$ getrocknet und eingeengt. Das kristalline Rohprodukt wird aus Acetonitril umkristallisiert, wobei 4,7 g der Titelverbindung anfallen.

$R_f$ (DIP) = 0,4; MS (DCI) = 411 (M + 1); Schmp. = 176 - 178 °C; $[\alpha]_D^{20}$ = +35,7 ° (c = 1, $CH_3OH$).

Beispiel 1c

(4S,5R,1'R)-3-t.-Butyloxycarbonyl-4-cyclohexylmethyl-2,2-dimethyl-5-[1'-benzyloxy-1'-(2-thiazolyl)methyl]-oxazolidin

5,4 g (13,2 mmol) der Titelverbindung 1b werden in 100 ml THF gelöst und zu 0,7 g Natriumhydrid (50 % in Öl, 2 x mit Hep gewaschen) in 20 ml THF getropft. Nach 20 min bei Rückfluß wird auf 50 °C abgekühlt und 0,5 g Tetrabutylammmoniumjodid und 1,76 ml Benzylbromid zugesetzt. Nach 3 Stunden Rühren bei RT wird in ca. 500 ml ges. NaCl-Lösung aufgenommen. Nach zweimaliger Extraktion mit Essigester wird mit $Na_2SO_4$ getrocknet und eingeengt, wobei 5 g der Titelverbindung anfallen, die ohne weitere Reinigung weiter umgesetzt werden können.
$R_f$ (DIP) = 0,45; MS (DCI) = 500 (M + 1); Schmp. = 96 °C;
$[\alpha]_D^{20}$ = +46,3 ° (c = 1, $CH_3OH$).

Beispiel 1d

(4S,5R,1'R)-3-t.-Butyloxycarbonyl-4-cyclohexylmethyl-2,2-dimethyl-5-(1'-benzyloxy-1'-formyl)-oxazolidin

2,1 g (4,2 mmol) der Titelverbindung 1c werden in 10 ml Acetonitril gelöst und 0,55 ml Dimethylsulfat zugesetzt. Nach 8 Stunden Rückfluß und Stehen über Nacht bei RT wird zur Trockne eingeengt. Das Rohprodukt wird in 15 ml $CH_3OH$ gelöst und bei -10 °C 0,5 g $NaBH_4$ in kleinen Portionen zugegeben und die Lösung wird eingeengt. Der Rückstand wird in EE aufgenommen, mit ges. NaCl-Lösung gewaschen und mit $Na_2SO_4$ getrocknet. Nach Einengen wird das Rohprodukt in 23 ml Aceton/$H_2O$ (95 : 5) gelöst und zu 2,25 g NBS in 22 ml Aceton/$H_2O$ (95 : 5) getropft (15 min, -10 °C). In kleinen Portionen werden 80 ml ges. $Na_2SO_4$-Lösung zugegeben, so daß die Temperatur nicht über +20 °C steigt. Nach Verdünnen mit 100 ml $H_2O$ wird 3 x mit $Et_2O$ extrahiert. Die vereinigten Extrakte werden 2 x mit $H_2O$ gewaschen, mit $MgSO_4$ getrocknet und eingeengt, wobei 1,75 g (95 %) der Titelverbindung anfallen, die als Rohprodukt weiterverwendet werden.
$R_f$ (DIP) = 0,4; MS (DCI) = 446 (M + 1).

Beispiel 1e

(4S,5R,1'S)-3-t.-Butyloxycarbonyl-4-cyclohexylmethyl-2,2-dimethyl-5-[1'-benzyloxy-3'-(2-pyridyl)-prop-2'-enyl]-oxazolidin

1,72 g (4 mmol) 2-Picolyltriphenylphosphoniumchlorid werden in 30 ml trockenem THF suspendiert. 0,43 g Kalium-t.-butylat werden unter Argon zugesetzt. Nach 2 Stunden bei RT werden 0,85 g der Titelverbindung 1d in 5 ml THF in die zitronengelbe Lösung getropft (-15 °C). Nach Stehen bei RT über Nacht wird mit $H_2O$ verdünnt, mit EE (3 x) extrahiert, mit ges. NaCl-Lösung gewaschen, getrocknet ($Na_2SO_4$) und eingeengt. Chromatographie an $SiO_2$ liefert 0,11 g cis-Isomer und 0,4 g trans-Isomer der Titelverbindung als Öl.

$$R_f \text{ (DIP)} = \begin{array}{l} 0{,}45 \text{ (cis)} \\ 0{,}3 \text{ (trans)}; \end{array}$$

MS (DCI) = 521 (M + 1);
$[\alpha]_D^{20}$ = +26,8 ° (c = 1, MeOH, trans)

**Patentansprüche**

1. Verbindung der Formel Ia oder Ib

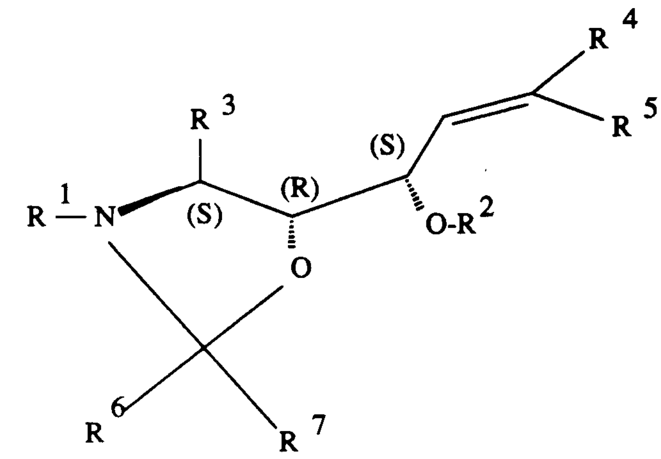

(Ia)

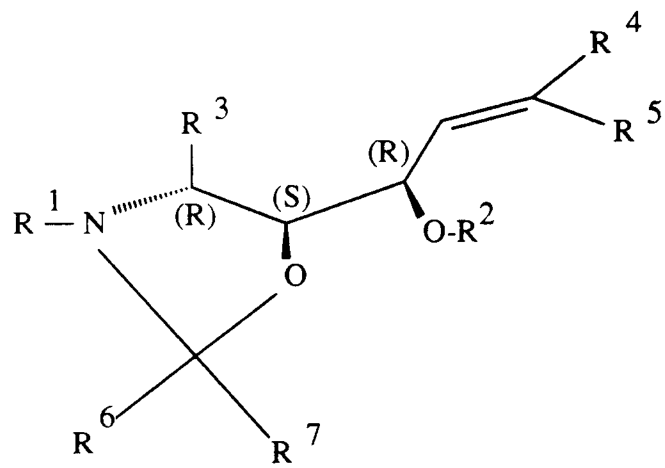

(Ib)

in weicher

$R^1$ $R^a$-W- bedeutet;

W für -CO-, -O-CO-, $-SO_2-$ oder -NH-CO- steht;

$R^a$ für Wasserstoff, $(C_1-C_{10})$-Alkyl, das gegebenenfalls ein- oder zweifach ungesättigt ist und das gegebenenfalls mit bis zu 3 gleichen oder verschiedenen Resten aus der Reihe Hydroxy, $(C_1-C_7)$-Alkoxy, $(C_1-C_7)$-Alkanoyloxy, Carboxy, $(C_1-C_7)$-Alkoxycarbonyl, Halogen, Amino, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino substituiert ist, $(C_3-C_8)$-Cycloalkyl, $(C_3-C_8)$-Cycloalkyl-$(C_1-C_6)$-alkyl, $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogenen substituiertes Anilino und Trifluormethyl substituiert ist, $(C_6-C_{14})$-Aryl-$(C_1-C_6)$-alkyl, worin der Arylteil gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl und Sulfo substituiert ist, oder den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom als Ringglieder, der gegebenenfalls durch einen oder mehrere Reste aus der Reihe F, Cl, Br, Hydroxy, $(C_1-C_7)$-Alkoxy, $(C_1-C_7)$-Alkyl, $(C_1-C_8)$-Alkoxycarbonyl, Amino und Trifluormethyl mono-, di- oder trisubstituiert ist, steht.

$R^2$ H oder eine Schutzgruppe der Hydroxylgruppe bedeutet;

$R^3$ bedeutet ($C_1$-$C_{12}$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_6$)-alkyl, wobei der Cycloalkylteil jeweils gegebenenfalls mit ($C_1$-$C_6$)-Alkyl substituiert ist.

$R^4$ und $R^5$ sind gleich oder verschieden und bedeuten Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_8$-$C_{14}$)-Aryl-($C_1$-$C_6$)-alkyl, wobei Aryl jeweils mit ein, zwei oder drei gleichen oder verschiedenen Resten aus der Gruppe ($C_1$-$C_6$)-Alkyl und Halogen substituiert sein kann, Het oder Het-($C_1$-$C_6$)-alkyl, wobei Het für einen 5-, 6- oder 7-gliedrigen heterocyclischen Ring steht, der gegebenenfalls benzanelliert ist und aromatisch, teilhydriert oder vollständig hydriert sein kann und der als Heteroelement ein oder zwei gleiche oder verschiedene Reste aus der Gruppe N, O, S, NO, SO und $SO_2$ enthält und der mit ein oder zwei gleichen oder verschiedenen Resten aus der Gruppe ($C_1$-$C_6$)-Alkyl, ($C_1$-$C_4$)-Alkoxy und Halogen substituiert sein kann; und

$R^6$ und $R^7$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl-($C_1$-$C_6$)-alkyl bedeuten, wobei Aryl jeweils mit ein, zwei oder drei gleichen oder verschiedenen Resten aus der Gruppe ($C_1$-$C_6$)-Alkyl, ($C_1$-$C_4$)-Alkoxy und Halogen substituiert sein kann; oder $R^6$ und $R^7$ zusammen mit dem sie tragenden Kohlenstoffatom ($C_3$-$C_{12}$)-Cycloalkyl bedeuten;

sowie deren Salze.

**2.** Verbindung der Formel Ia oder Ib, in welcher

$R^a$ für Wasserstoff, ($C_1$-$C_{10}$)-Alkyl, das gegebenenfalls ein- oder zweifach ungesättigt ist, ($C_3$-$C_8$)-Cycloalkyl, ($C_3$-$C_8$)-Cycloalkyl-($C_1$-$C_6$)-alkyl, ($C_6$-$C_{14}$)-Aryl, ($C_6$-$C_{14}$)-Aryl-($C_1$-$C_6$)-alkyl, oder für den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom auch als Ringglieder steht,

$R^3$ ($C_1$-$C_6$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_3$)-alkyl bedeutet;

$R^6$ und $R^7$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl bedeuten; oder diese zusammen mit dem sie tragenden Kohlenstoffatom für ($C_3$-$C_{12}$)-Cycloalkyl stehen;

und die übrigen Reste wie in Anspruch 1 definiert sind;

sowie deren Salze.

**3.** Verbindung der Formel Ia oder Ib gemäß Anspruch 1 oder 2,

in welcher

W für -CO-, -O-CO- steht;

$R^a$ Wasserstoff, ($C_1$-$C_{10}$)-Alkyl, das gegebenenfalls ein- oder zweifach ungesättigt ist, ($C_3$-$C_8$)-Cycloalkyl, ($C_3$-$C_8$)-Cycloalkyl-($C_1$-$C_6$)-alkyl, ($C_6$-$C_{14}$)-Aryl, ($C_6$-$C_{14}$)-Aryl-($C_1$-$C_6$)-alkyl, oder den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom als Ringglieder bedeutet;

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;

$R^6$ und $R^7$ gleich sind und Wasserstoff oder ($C_1$-$C_4$)-Alkyl bedeuten, oder diese zusammen mit dem sie tragenden Kohlenstoffatom für ($C_5$-$C_7$)-Cycloalkyl stehen;

und die übrigen Reste wie in Anspruch 1 definiert sind;

sowie deren Salze.

**4.** Verbindung der Formel Ia oder Ib gemäß einem der Ansprüche 1 bis 3,

in welcher

$R^1$ und $R^3$ wie in Anspruch 3 definiert sind;

$R^4$ und $R^5$ gleich oder verschieden sind und Wasserstoff, $(C_1-C_4)$-Alkyl, $(C_5-C_7)$-Cycloalkyl, $(C_5-C_7)$-Cycloalkyl-$(C_1-C_3)$-alkyl, Phenyl, Benzyl, 2-Pyridyl, 3-Pyridyl, 4-Pyridyl, 2-Thiazolyl, 4-Thiazolyl, 5-Thiazolyl, 2-Thienyl, 3-Thienyl, 1-Methylimidazol-2-yl, 1-Methylimidazol-4-yl oder 1-Methylimidazol-5-yl bedeuten;

$R^6$ und $R^7$ gleich sind und Wasserstoff, $(C_1-C_4)$-Alkyl oder zusammen mit dem sie tragenden Kohlenstoffatom ein $(C_5-C_7)$-Cycloalkyl bedeuten;

und die übrigen Reste wie in Anspruch 1 definiert sind;

sowie deren Salze.

**5.** Verfahren zur Herstellung von Verbindungen der Formel Ia und Ib gemäß einem der Ansprüche 1 bis 4, dadurch gekennzeichnet, daß man eine Verbindung der Formel IIa oder IIb

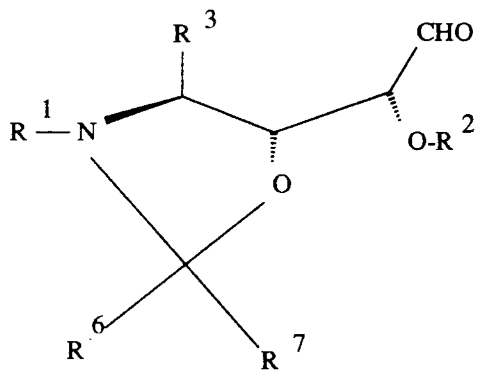

(IIa)

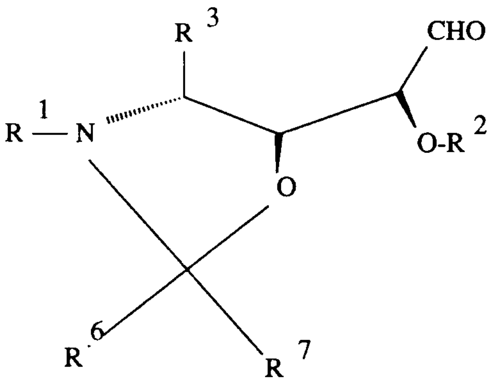

(IIb)

in welcher die Reste $R^1$, $R^2$, $R^3$, $R^6$ und $R^7$ wie in Anspruch 1 definiert sind, umsetzt mit einem Phosphoran der Formel III,

in welcher $R^4$ und $R^5$ wie in Anspruch 1 definiert sind und $R^8$, $R^9$ und $R^{10}$ für gleiche oder verschiedene Arylreste steht,

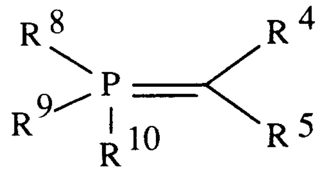

(III)

und die erhaltenen Verbindungen gegebenenfalls in ihr Salz überführt.

**6.** Verbindung der Formel IIa oder IIb

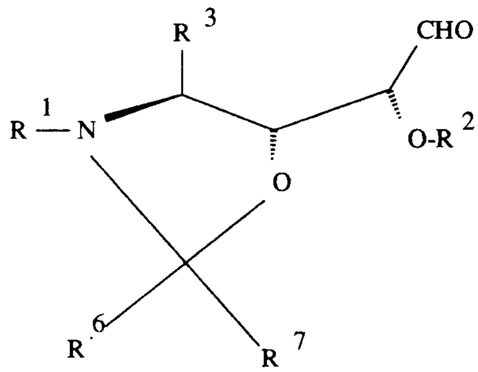

(IIa)

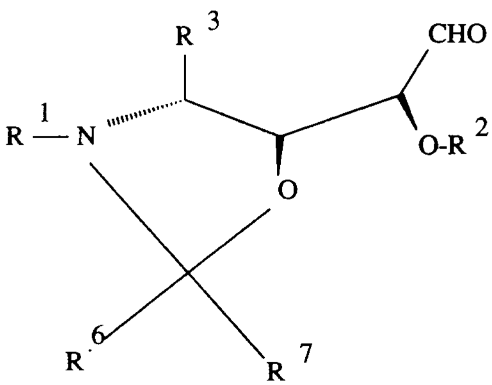

(IIb)

in welcher $R^1$, $R^2$, $R^3$, $R^6$ und $R^7$ wie in Anspruch 1 definiert sind.

**7.** Verwendung einer Verbindung gemäß einem der Ansprüche 1 bis 4 bei der Herstellung von Inhibitoren des Renins und der HIV-Protease.

**Patentansprüche für folgende Vertragsstaaten : ES, GR**

**1.** Verfahren zur Herstellung einer Verbindung der Formel (Ia) oder (Ib)

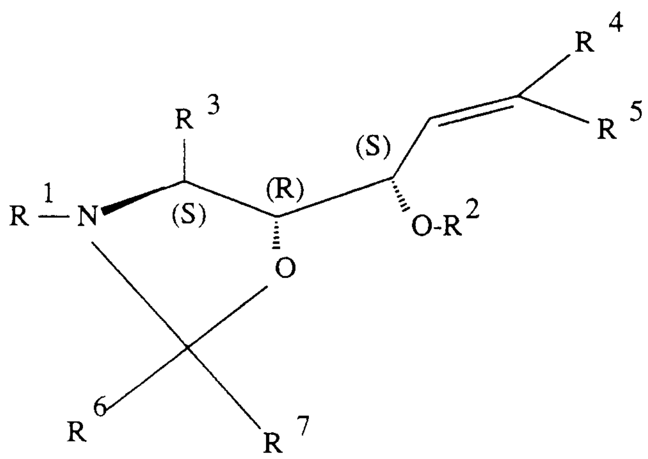

(Ia)

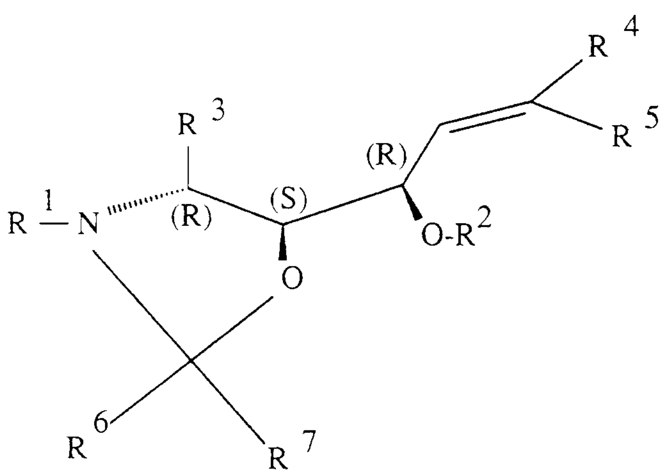

(Ib)

in welcher

$R^1$ $R^a$-W- bedeutet;

W für -CO-, -O-CO-, $-SO_2-$ oder -NH-CO- steht;

$R^a$ für Wasserstoff, $(C_1-C_{10})$-Alkyl, das gegebenenfalls ein- oder zweifach ungesättigt ist und das gegebenenfalls mit bis zu 3 gleichen oder verschiedenen Resten aus der Reihe Hydroxy, $(C_1-C_7)$-Alkoxy, $(C_1-C_7)$-Alkanoyloxy, Carboxy, $(C_1-C_7)$-Alkoxycarbonyl, Halogen, Amino, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino substituiert ist, $(C_3-C_8)$-Cycloalkyl, $(C_3-C_8)$-Cycloalkyl-$(C_1-C_6)$-alkyl, $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogenen substituiertes Anilino und Trifluormethyl substituiert ist, $(C_6-C_{14})$-Aryl-$(C_1-6_6)$-alkyl, worin der Arylteil gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl und Sulfo substituiert ist, oder den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom als Ringglieder, der gegebenenfalls durch einen oder mehrere Reste aus der Reihe F, Cl, Br, Hydroxy, $(C_1-C_7)$-Alkoxy, $(C_1-C_7)$-Alkyl, $(C_1-C_8)$-Alkoxycarbonyl, Amino und Trifluormethyl mono-, di- oder trisubstituiert ist, steht;

$R^2$ H oder eine Schutzgruppe der Hydroxylgruppe bedeutet;

$R^3$ bedeutet ($C_1$-$C_{12}$)-Alkyl, mono-, bi- oder tricyclisches ($C_3$-$C_{18}$)-Cycloalkyl oder mono-, bi- oder tricyclisches ($C_3$-$C_{18}$)-Cycloalkyl-($C_1$-$C_6$)-alkyl, wobei der Cycloalkylteil jeweils gegebenenfalls mit ($C_1$-$C_6$)-Alkyl substituiert ist.

$R^4$ und $R^5$ sind gleich oder verschieden und bedeuten Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)alkyl, ($C_8$-$C_{14}$)-Aryl-($C_1$-$C_6$)-alkyl, wobei Aryl jeweils mit ein, zwei oder drei gleichen oder verschiedenen Resten aus der Gruppe ($C_1$-$C_6$)-Alkyl und Halogen substituiert sein kann, Het oder Het-($C_1$-$C_6$)-alkyl, wobei Het für einen 5-, 6- oder 7-gliedrigen heterocyclischen Ring steht, der gegebenenfalls benzanelliert ist und aromatisch, teilhydriert oder vollständig hydriert sein kann und der als Heteroelement ein oder zwei gleiche oder verschiedene Reste aus der Gruppe N, O, S, NO, SO und $SO_2$ enthält und der mit ein oder zwei gleichen oder verschiedenen Resten aus der Gruppe ($C_1$-$C_6$)-Alkyl, ($C_1$-$C_4$)-Alkoxy und Halogen substituiert sein kann; und

$R^6$ und $R^7$ gleich oder verschieden sind und Wasserstoff, ($C_1$-$C_{12}$)-Alkyl, ($C_3$-$C_{12}$)-Cycloalkyl, ($C_3$-$C_{12}$)-Cycloalkyl-($C_1$-$C_6$)-alkyl, ($C_6$-$C_{14}$)-Aryl-($C_1$-$C_6$)-alkyl bedeuten, wobei Aryl jeweils mit ein, zwei oder drei gleichen oder verschiedenen Resten aus der Gruppe ($C_1$-$C_6$)-Alkyl, ($C_1$-$C_4$)-Alkoxy und Halogen substituiert sein kann; oder $R^6$ und $R^7$ zusammen mit dem sie tragenden Kohlenstoffatom ($C_3$-$C_{12}$)-Cycloalkyl bedeuten;
sowie deren Salze, dadurch gekennzeichnet, daß man eine Verbindung der Formel (IIa) oder (IIb)

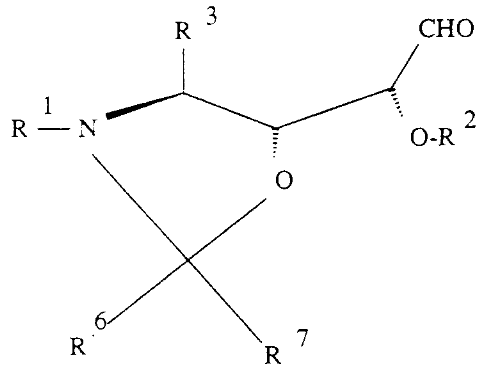

(IIa)

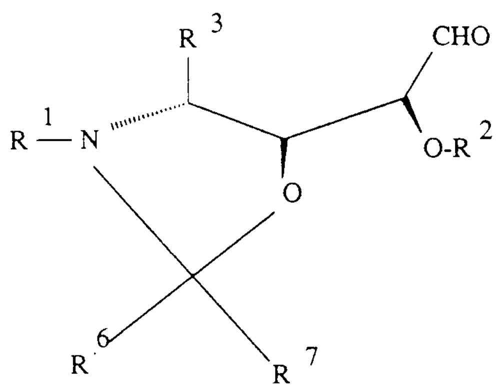

(IIb)

in welcher die Reste $R^1$, $R^2$, $R^3$, $R^6$ und $R^7$ wie oben definiert sind, umsetzt mit einem Phosphoran der Formel (III), in welcher $R^4$ und $R^5$ wie oben definiert sind und $R^8$, $R^9$ und $R^{10}$ für gleiche oder verschiedene Arylreste steht,

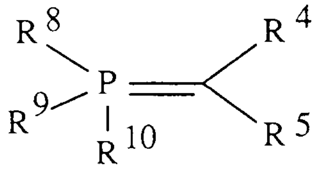

(III)

und die erhaltenen Verbindungen gegebenenfalls in ihr Salz überführt.

**2.** Verfahren gemäß Anspruch 1, dadurch gekennzeichnet, daß man eine Verbindung der Formel (Ia) oder (Ib) herstellt, in welcher

$R^a$ für Wasserstoff, $(C_1-C_{10})$-Alkyl, das gegebenenfalls ein- oder zweifach ungesättigt ist, $(C_3-C_8)$-Cycloalkyl, $(C_3-C_8)$-Cycloalkyl-$(C_1-C_6)$-alkyl, $(C_6-C_{14})$-Aryl, $(C_6-C_{14})$-Aryl-$(C_1-C_6)$-alkyl, oder für den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom auch als Ringglieder steht,

$R^3$ $(C_1-C_6)$-Alkyl, mono-, bi- oder tricyclisches $(C_3-C_{18})$-Cycloalkyl oder mono-, bi- oder tricyclisches $(C_3-C_{18})$-Cycloalkyl-$(C_1-C_3)$-alkyl bedeutet;

$R^6$ und $R^7$ gleich oder verschieden sind und Wasserstoff, $(C_1-C_{12})$-Alkyl, $(C_3-C_{12})$-Cycloalkyl, $(C_3-C_{12})$-Cycloalkyl-$(C_1-C_6)$-alkyl bedeuten; oder diese zusammen mit dem sie tragenden Kohlenstoffatom für $(C_3-C_{12})$-Cycloalkyl stehen;

und die übrigen Reste wie in Anspruch 1 definiert sind;

sowie deren Salze.

**3.** Verfahren gemäß Anspruch 1, dadurch gekennzeichnet, daß man eine Verbindung der Formel (Ia) oder (Ib) herstellt, in welcher

W für -CO-, -O-CO- steht;

$R^a$ Wasserstoff, $(C_1-C_{10})$-Alkyl, das gegebenenfalls ein- oder zweifach ungesättigt ist, $(C_3-C_8)$-Cycloalkyl, $(C_3-C_8)$-Cycloalkyl-$(C_1-C_6)$-alkyl, $(C_6-C_{14})$-Aryl, $(C_6-C_{14})$-Aryl-$(C_1-C_6)$-alkyl, oder den Rest eines 5- oder 6-gliedrigen monocyclischen oder 9- oder 10-gliedrigen bicyclischen Heterocyclus mit mindestens 1 C-Atom, 1 bis 4 N-Atomen und/oder 1 S- oder O-Atom als Ringglieder bedeutet;

$R^3$ Methyl, Ethyl, n-Propyl, n-Butyl, 2-Methylpropyl, 2-Ethylbutyl, Cyclopentylmethyl, Cyclohexylmethyl oder Cycloheptylmethyl bedeutet;

$R^6$ und $R^7$ gleich sind und Wasserstoff oder $(C_1-C_4)$-Alkyl bedeuten, oder diese zusammen mit dem sie tragenden Kohlenstoffatom für $(C_5-C_7)$-Cycloalkyl stehen;

und die übrigen Reste wie in Anspruch 1 definiert sind;

sowie deren Salze.

**4.** Verfahren gemäß Anspruch 1, dadurch gekennzeichnet, daß man eine Verbindung der Formel (Ia) oder (Ib) herstellt, in welcher

$R^1$ und $R^3$ wie in Anspruch 3 definiert sind;

$R^4$ und $R^5$ gleich oder verschieden sind und Wasserstoff, $(C_1-C_4)$-Alkyl, $(C_5-C_7)$-Cycloalkyl, $(C_5-C_7)$-Cycloalkyl-$(C_1-C_3)$-alkyl, Phenyl, Benzyl, 2-Pyridyl, 3-Pyridyl, 4-Pyridyl, 2-Thiazolyl, 4-Thiazolyl, 5-Thiazolyl, 2-Thienyl, 3-Thienyl, 1-Methylimidazol-2-yl, 1-Methylimidazol-4-yl oder 1-Methylimidazol-5-yl bedeuten;

$R^6$ und $R^7$ gleich sind und Wasserstoff, $(C_1\text{-}C_4)$-Alkyl oder zusammen mit dem sie tragenden Kohlenstoffatom ein $(C_5\text{-}C_7)$-Cycloalkyl bedeuten;

und die übrigen Reste wie in Anspruch 1 definiert sind;

sowie deren Salze.

**5.** Verwendung einer Verbindung der Formel (IIa) oder (IIb)

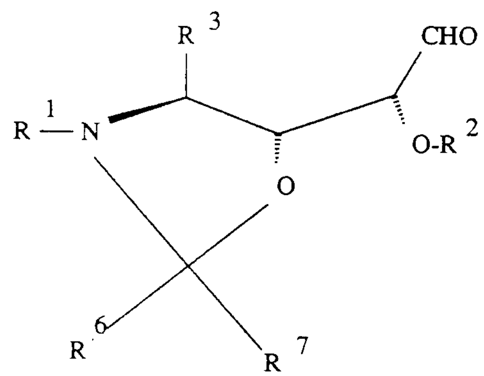

(IIa)

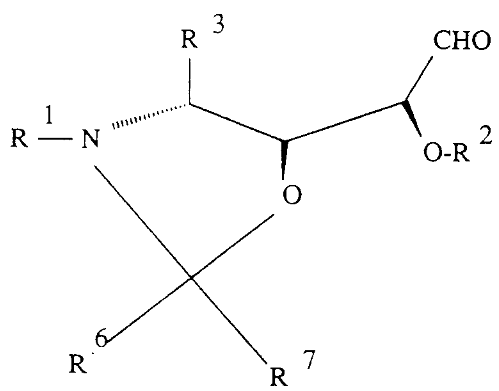

(IIb)

in welcher $R^1$, $R^2$, $R^3$, $R^6$ und $R^7$ wie in Anspruch 1 definiert sind, zur Herstellung einer Verbindung der Formel (Ia) oder (Ib) gemäß Anspruch 1.

**6.** Verwendung einer Verbindung der Formel (Ia) oder (Ib), hergestellt gemäß einem der Ansprüche 1 bis 4 bei der Herstellung von Inhibitoren des Renins und der HIV-Protease.

Europäisches Patentamt

# EUROPÄISCHER RECHERCHENBERICHT

Nummer der Anmeldung

EP 91 11 9397

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-0 329 295 (THE UPJOHN COMPANY)<br>* Seite 28, Zeile 41 - Seite 29, Zeile 6 *<br>* Seite 69 *<br>----- | 1-4,7 | C07D263/06<br>C07D413/06 |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 12 FEBRUAR 1992 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)